# NEW EUROPEAN PATENT SPECIFICATION

(11) **EP 2 552 431 B2**
(45) Date of publication and mention of the opposition decision: **12.08.2026**
(45) Mention of the grant of the patent: 14.06.2017
(21) Application number: 11717046.4
(22) Date of filing: 28.03.2011
(51) Int. Cl.: A61K 31/05, A61K 45/06, A61P 31/00, A61P 31/04, A61P 31/12

(54) **CARVACROL/THYMOL COMPOSITION FOR THE TREATMENT OF INFECTIOUS SALMON ANEMIA**
CARVACROL/THYMOL ZUSAMMENSETZUNGEN FÜR DIE BEHANDLUNG VON INFEKTIÖSER LACHSANÀMIE
COMPOSITION DU CARVACRL/THYMOL POUR LE TRAITEMENT DE L'ANEMIE INFECTUEUSE DU SALMON

(30) Priority: 26.03.2010 NO 20100454
(43) Date of publication of application: 06.02.2013
(73) Proprietor: Ewos Innovation AS, 4335 Dirdal (NO)
(72) Inventor: TRANCOSO KIRSTEN, José, Miguel, Puerto Varas Región de Los Lagos (CL); PINO MARAMBIO, Jorge, Eduardo, Región de Los Lagos (CL); GONZÁLEZ FORETIC, Nestor, Javier, Casilla 1219 Puerto Varas X Región de Los Lagos (CL); GONZÁLEZ VECINO, José, Luis, N-4013 Stavanger (NO); EL-MOWAFI, Adel, N-4308 Sandnes (NO)
(74) Representative: Strych, Sebastian
(86) International application number: PCT/NO2011/000106
(87) International publication number: WO 2011/119047

(56) References cited:
- WO-A1-2007/090714
- WO-A2-2004/091307
- JP-A- H02 207 758
- O JINTASATAPORN, C KAMEL: "THE EFFECT OF THE FEEDING OF ENCAPSULATED THYMOL AND CARVACROL IN LOW-PROTEIN DIETS FOR HYBRID CATFISH ON PERFORMANCE UNDER NORMAL AND DISEASE CHALLENGE CONDITIONS", 5 August 2009 (2009-08-05), XP002646013, Retrieved from the Internet <URL:http://en.engormix.com/MA-aquaculture/articles/the-effect-feeding-encapsulated-t1391/p0.htm> [retrieved on 20110629]
- DATABASE BIOSIS [online] BIOSCIENCES INFORMATION SERVICE, PHILADELPHIA, PA, US; HEIL T P ET AL: "SENSORY PROPERTIES OF THIOPHENOLS AND ALKYLPHENOLS CAUSING FLAVOR TAINTING IN FISH FROM THE UPPER WISCONSIN RIVER USA", XP002427057, retrieved from BIOSIS Database accession no. 198988125920

## Description

### Field of the invention

The present invention relates to a propyl-methyl-phenol compound or composition comprising a propyl-methyl-phenol compound for use in the treatment and/or prophylaxis of Infectious salmon anemia (ISA) and/or diseases caused by Infectious salmon anemia virus.

### Salmonid rickettsial septicemia (SRS), pathogen agent Piscirickettsia salmonis

Salmonid rickettsial septicemia (SRS), also known as piscirickettsiosis, is a fatal disease in salmonids. Although the etiological agent for SRS was identified in the late 1980's as *Piscirickettsia salmonis,* antibiotics proved to be an unsuccessful treatment, due, at least in part, to the intracellular nature of this bacterium. As a consequence of the lack of a viable treatment, millions of farmed salmon die of SRS each year just in southern Chile.

*Piscirickettsia salmonis* is a small, intracellular bacterium that causes a fatal septicaemic condition of salmonids. Since initial isolation in the late 1980's, *P. salmonis* has been the primary cause of mortality of the industry in Chile. During 1995 more than 10 million salmon died during marine farming operations. Most of the losses were attributed to *P. salmonis,* with the economic impact estimated at $49 million USD. Because *P. salmonis* is intracellular (lives inside the cells of the host), efficacy of antibiotic treatment of infected salmon is poor. Vaccine development has also proved difficult due to the intracellular nature of the organism, the different strains of *P*. *salmonis* and the lack immunological memory in salmonids. Antimicrobial use, such as oxolinic acid, has been extensive for a number of years in Chile reaching 127 tonnes (active compound) by 2003. No antibiotic or chemotherapeutant has proved to be consistently effective against *P*. *salmonis* and increasing resistance encountered. The development of widespread antibiotic resistance would cause significant operational problems and a major financial impact on the industry.

### Infectious salmon anemia, and Infectious salmon anemia virus (ISAv)

Infectious Salmon Anaemia (ISA) is a double stranded RNA virus belonging to the *Orthomyxoviridae.* The virus has caused severe economic impact in many of the salmon producing regions including Chile, Norway, Scotland, Faroes, Canada and USA. The disease results in severe anaemia in Atlantic salmon, affecting the haematopoietic tissues including the spleen, kidney as well as the liver and heart. Trout and other marine species may be asymptomatic carriers of the disease. Levels of mortality are highly variable, but exceptional losses of up to 90% have been recorded. The peaks of outbreak generally occur in the spring and early summer with temperatures around 10°C. ISA has occurred within a temperature range of 3°C - 15°C. In 1997 ISA was listed in the European Union Directive 91/67/EC classified the disease as a "List I disease".

Infectious salmon anemia or anaemia (ISA) is a viral disease of Atlantic Salmon (*Salmo salar*) that affects fish farms in Canada, Norway, Scotland and Chile, causing severe losses to infected farms.

As the name implies, it causes severe anemia of infected fish. Unlike mammals, the red blood cells of fish have DNA, and can become infected with viruses. The fish develop pale gills, and may swim close to the water surface, gulping for air. However, the disease can also develop without the fish showing any external signs of illness, the fish maintain a normal appetite, and then they suddenly die. The disease can progress slowly throughout an infected farm and, in the worst cases, death rates may approach 100%. Post-mortem examination of the fish has shown a wide range of causes of death. The liver and spleen may be swollen, congested or partially already dead. The circulatory system may stop working, and the blood may be contaminated with dead blood cells. Red blood cells still present often burst easily and the numbers of immature and damaged blood cells are increased.

Infectious salmon anemia appears to be most like influenza viruses. Its mode of transfer and the natural reservoirs of infectious salmon anemia virus are not fully understood. Apart from Atlantic salmon, both sea-run Brown trout (*Salmo trutta*) and Rainbow trout (*Oncorhynchus mykiss*) can be infected, but do not become sick, so it is thought possible that these species may act as important carriers and reservoirs of the virus.

The virus can survive in seawater and, not surprisingly, a major risk factor for any uninfected farm is its proximity to an already infected farm.

More recently the sea louse, a small crustacean parasite that attacks the protective mucous, scales and skin of the salmon has been shown to carry the virus passively on its surface and in its digestive tract, although transmission of the disease by sea lice has not been demonstrated.

It is therefore very important to find new compounds and ways to treat and prevent *Piscirickettsia salmonis* and ISAv caused diseases in fish.

The objective technical problem of the present invention is to provide new effective agents and methods for killing or regulating ISAv.

### Summary of the invention

A first aspect of the present invention relates to a propyl-methyl-phenol compound, or composition comprising a propyl-methyl-phenol compound for use in the treatment and/or prophylaxis of Infectious salmon anemia (ISA) and/or diseases caused by Infectious salmon anemia virus.

Preferred embodiments of the first aspect of the present invention are given in the subclaims 2-10.

A second aspect of the present invention relates to a use of a propyl-methyl-phenol compound, or composition comprising a propyl-methyl-phenol compound for the manufacturing of a pharmaceutical and/or nutraceutical composition the treatment and/or prophylaxis of Infectious salmon anemia (ISA) and/or diseases caused by Infectious salmon anemia virus.

Preferred embodiments of the second aspect of the present invention are given in the subclaims 2-10.

Also described herein is a fish feed comprising conventional feed ingredients such as proteins, lipids, carbohydrates, vitamins and minerals, characterized in that said fee also comprises Carvacrol and Thymol (not according to the invention).

The ratio of Carvacrol:Thymol can be about 1:1.

Carvacrol and Thymol can be present in the fish feed in an amount of about 15 ppm Carvacrol and 15 ppm Thymol.

### Detailed description of the invention

Figure 1 shows the observed survival of Rainbow trout with an outbreak of SRS given various treatments as described in example 3. Each curve denotes a cage. "Control" is a commercial feed (EWOS Silva), whereas "BG" is a boost premix, and "BG + new" is the boost premix and 15 ppm of Carvacrol and 15 ppm of Thymol.
Figure 2 which shows the estimated hazard ratios (relative risk of death) in relation to the control feed (risk taken as 1 and marked with the dashed red horizontal line). Error bars show 95% credible intervals. "BG" is a boost premix, and "BG + new" is the boost premix and 15 ppm of Carvacrol and 15 ppm of Thymol.
Figure 3 shows the CT values for *P. salmonis* in brain tissues at the initiation of the trial described in example 3 obtained through qPCR.
Figure 4 shows the CT values for *P. salmonis* in brain tissues at the end of the trial described in example 3 obtained through qPCR.
Figure 5 shows the CT values for *P. salmonis* in kidney tissues at the initiation of the trial described in example 3 obtained through qPCR.
Figure 6 shows the CT values for *P. salmonis* in kidney tissues at the end of the trial described in example 3 obtained through qPCR.

### Detailed description of the invention

### Experimental section

### Example 1 - Effect of Carvacrol on Piscirickettsia salmonis (not according to the invention)

The objective of the present study is to evaluate the efficacy of Carvacrol, and a combination of Carvacrol and Thymol against *Piscirickettsia salmonis* (*P. salmonis*) by the *in vitro* determination of Minimum Inhibitory Concentration (MIC).

### Materials and Methods

### Preparation of the tested compounds

The preparation of the working solutions of Carvacrol and Thymol was performed by the Laboratory of Ecological Chemistry, Faculty of Chemistry, Universidad de La Frontera, Temuco, Chile. Carvacrol (Sigma Aldrich 98% standard grade) was diluted in distilled water/ethanol solution up to 200 ppm (termed "CAR1 "). The Carvacrol + Thymol (Sigma Aldrich 98% standard grade) 1:1 (volume:volume) were diluted in DMSO / distilled water at 200 ppm (termed "CAR2"). All products, stock solution and working solution were stored under refrigeration (4° C) and were handled under laminar flow chamber in order to avoid contamination of the products to evaluate.

### Antibacterial efficacy

The evaluation of the MIC of the different solutions of Carvacrol and Thymol against *P*. *salmonis* was performed at ADL Diagnostics, Puerto Montt, Chile. Six different isolates of *P*. *salmonis* were obtained from clinical cases received at ADL Diagnostics and grown at 18 °C in agar PSA1, specific for *P*. *salmonis.* The different isolates were confirmed by TagMan^{®} RT-PCR (A. Casanova, J. Obreque C., A. M. Sandino G. and M. Jashés. 2001. tRNA genes were found in Piscirickettsia salmonis 16S-23S rDNA spacer region (ITS). FEMS Microbiology Letters. Vol. 197: 35 19-22) (http://www3.appliedbiosystems.com/). Isolates were labelled as: Ps1: PM-7 10908; Ps2: PM-10877; Ps3: PM-10795; Ps4: PM-10766; Ps5: PM-10727 and Ps6: PM-10707.

### Minimum Inhibitory Concentration (MIC)

The MIC for the six isolates was determined using a standardized method of the National Comitee for Clinical Laboratory Standards (NCCLS, 2005) based on standard M49-P (2005), which involves carrying out serial dilutions of the product (100; 50; 25; 12.5; 6.25; 3.1; 1.5; 0.7; 0.35; 0.18; 0.09 ppm) up to obtain the minimal concentration that inhibits bacterial growth.

Inoculums from pure isolates in agar PSA were obtained to achieve a concentration of 0.5 McF, which were planted with the different dilutions of the test compounds to evaluate antibacterial effect in culture microplates. The microplates were incubated at 18 °C for 24 h. Subsequently a peal was conducted with each dilution on an agar plate PSA, which were then incubated for 10 days to observe the growth of *P*. *salmonis.*

### Results and Discussion

The MIC for Carvacrol (CAR1) reached a value in the range of 3.7 to 7.4 ppm. For the particular case of Thymol (CAR2) the MIC achieved was of 3.7 ppm in all isolates tested, indicating that Thymol would enhance the antibacterial action of Carvacrol alone (Table 1).

**Table 1.**

| Minimum Inhibitory Concentration (MIC) of Carvacrol (CAR1) and Carvacrol + Thymol (CAR1 + CAR2) against *P. salmonis* isolates. | | | | | | |
|---|---|---|---|---|---|---|
| | PS1 | PS2 | PS3 | PS4 | PS5 | PS6 |
| | [MIC] ppm | | | | | |
| CAR1 | 7.4 | 7.4 | 7.4 | 3.7 | 7.4 | 3.7 |
| CAR1 + CAR2 | 3.7 | 3.7 | 3.7 | 3.7 | 3.7 | 3.7 |

The following conclusions could be obtained from this experiment:
1. Carvacrol inhibits the growth population of *P. salmonis* in an *in vitro* study.
2. The estimated MIC for Carvacrol was between 3.7 and 7.4 ppm
3. The estimated MIC for Carvacrol and Thymol was about 3.7 ppm, indicating that Thymol enhances the antibacterial action.

### Example 2 - the antiviral effect of Carvacrol and Thymol against ISAv.

ISAv is a serious problem for Chilean salmon industry with a high record of mortalities in the last two years.

The objective of the present study is to assess the antiviral efficacy of Carvacrol and Thymol against ISAv.

### Materials and Methods

### Preparation of the products

The preparation of the working solutions of Carvacrol was performed by the Laboratory of Ecological Chemistry, Faculty of Chemistry, Universidad de La Frontera, Temuco, Chile. Carvacrol (Sigma Aldrich 98% standard grade) was diluted in distilled water/ethanol solution up to 200 ppm (termed "CAR1 "). All products, stock solution and working solution were stored under refrigeration (4° C) and were handled under laminar flow chamber in order to avoid contamination of the products to evaluate.

### Strain of ISAv

It was used a strain of ISAv obtained by ADL Diagnostic (ADL-PM3205-ISAV-07), that was isolated from a kidney of Atlantic salmon (ASK-1) infested with ISAv in Isla Lemuy, Chiloé, Chile. The virus isolated was confirmed, through Indirect Immunofluorescence (IFAT) and RT-PCR.

### Challenge with ISAv

Eleven dilutions were prepared with Carvacrol (100, 50, 25, 12.5, 6.25, 3.1, 1.5, 0.7, 0.35, 0.18, 0.09 mg/L). Each dilution was challenge with ISAv in a concentration of 10^{7.25} TCID50 (Median tissue culture infective dose; that amount of a pathogenic agent that will produce pathological change in 50% of cell cultures inoculated), during 30, 60, 120 and 360 minutes. Then, each dilution of Carvacrol with ISAv was inoculated in a monolayer of ASK-1 cells (Atlantic salmon Kindey-1 cells, isolated and maintained in ADL Diagnostic) and incubated 10 days. The presence of ISAv in each layer of cells was determined, through IFAT (indirect fluorescent antibody test).

### Results

Carvacrol showed an effect against ISAv in the high dilution (100 mg/L) tested *in vitro,* after the virus adsorption (Table 2). After 60 minutes, Carvacrol inhibited the replication of the ISAv in all the replicas.

**Table 2**

| Absence (-) or Presence (+) of ISAv in tissue ASK-1 challenged with the virus and treated with different doses of Carvacrol (CAR 1) | | | | | | | | | | | | | | | | | |
|---|---|---|---|---|---|---|---|---|---|---|---|---|---|---|---|---|---|
| Dilution | | **CAR1** | | | | | | | | | | | | | | | |
| | | 30 minutes | | | | 60 minutes | | | | 120 minutes | | | | 360 minutes | | | |
| **No.** | **ppm** | 1 | 2 | 3 | 4 | 1 | 2 | 3 | 4 | 1 | 2 | 3 | 4 | 1 | 2 | 3 | 4 |
| 1 | 100 | + | - | - | + | - | - | - | - | - | - | - | - | - | - | - | - |
| 2 | 50 | + | + | + | + | + | + | + | + | + | + | + | + | + | + | + | + |
| 3 | 25 | + | + | + | + | + | + | + | + | + | + | + | + | + | + | + | + |
| 4 | 12,5 | + | + | + | + | + | + | + | + | + | + | + | + | + | + | + | + |
| 5 | 6,25 | + | + | + | + | + | + | + | + | + | + | + | + | + | + | + | + |
| 6 | 3,1 | + | + | + | + | + | + | + | + | + | + | + | + | + | + | + | + |
| 7 | 1,5 | + | + | + | + | + | + | + | + | + | + | + | + | + | + | + | + |
| 8 | 0,7 | + | + | + | + | + | + | + | + | + | + | + | + | + | + | + | + |
| 9 | 0,3 | + | + | + | + | + | + | + | + | + | + | + | + | + | + | + | + |
| 10 | 0,1 | + | + | + | + | + | + | + | + | + | + | + | + | + | + | + | + |
| 11 | 0,09 | + | + | + | + | + | + | + | + | + | + | + | + | + | + | + | + |

The following conclusions could be obtained from this experiment:
1. Carvacrol proved to eliminate the ISAv before the adsorption
2. The estimated dilution where Carvacrol eliminates ISAv is 100 ppm, after 60 minutes of challenge with the virus.

### Example 3 - The effect of Carvacrol and Thymol against Piscirickettsia salmonis in rainbow trout during onqrowing phase (not according to the invention)

The objects of this study are to:
1. 1) Asses the efficacy of Carvacrol and Thymol against *Piscirickettsia salmonis* in Rainbow trout (*O. mykiss*).
2. 2) Evaluate if the pulse treatments with Carvacrol and Thymol has an effect on mortality related with a *P. salmonis* outbreak.

Between November 2009 and December 2009 a trial was performed in Caniglia sea site of Mainstream (XI Region, Chile). Caniglia site had 16 cages of 30 m x 30 m with an automatic feeding system. Each cage contained close to 50,000 fish during the grow-out period. In Caniglia trial Rainbow trout (*O*. *mykiss*) were used in all cages from fish weight 1,280 g to 2,240 g.

In the trial, fish were fed a commercial feed (EWOS Silva) as control feed. For each treatment the same commercial feed was used as base but supplemented with: i) a dose of 15 ppm Carvacrol and 15 ppm Thymol plus boost premix (BG+new) and ii) only boost premix (BG). The boost premix used contained immunestimulant, vitamin C and prebiotic (0.5% of the formulation).

During the trial period, an outbreak of SRS (Salmonid Rickettsial Septicaemia) was observed since November 13^{th}. In order to mitigate its consequences, it was decided a treatment with functional feeds (BG and BG+new) and then compared with a control group. The trial was performed as a pulse of 21 days feeding, starting in November 26^{th}.

Each day was dead fish taken from the cages to be analyzed directly for the possible causes of mortality, and then recorded as cumulative mortality of the sea site. At the start and at the end of the pulse (November 26^{th} and December 17^{th}, respectively) samples of brain and kidney were taken from 10 moribund fish per cage. The samples were fixed in ethanol and tissues were analyzed by qPCR by the the PCR Laboratory of EWOS *Innovation* Chile (X Region, Chile). Relative concentrations of DNA present during the exponential phase of the reaction were determined by plotting fluorescence against cycle number on a logarithmic scale, where the cycle at which the fluorescence from a sample crosses the threshold was called the cycle threshold (CT).

Survival data was available from all cages and these data were first converted to a suitable form for survival analysis by adding the number of survived individuals at the end of the monitoring period with a "survived" status (censoring). All mortalities were given a "dead" status. Then the effect of the treatments on the survivorship was modeled with a multilevel Cox proportional hazard model (kinship package of R). Antilogs of the Cox model fixed effect parameters can be interpreted as hazard ratios. The credible intervals of the hazard ratios were computed by posterior simulation assuming multivariate normal distribution of the parameters with the estimated joint variance-covariance matrix and exponentiation the posterior distributions before taking the antilogarithms. The cage-to-cage variance was not considered in this simulation.

### Results

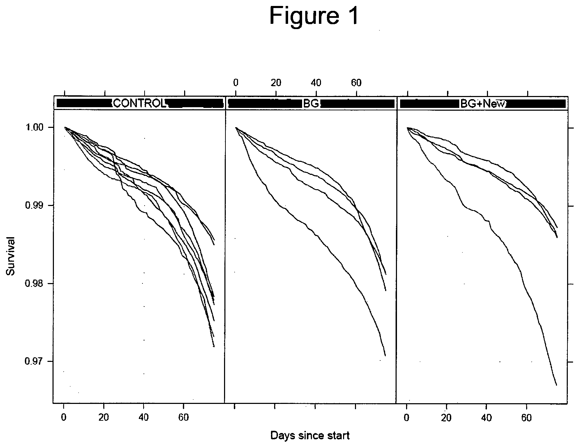

Feed consumptions between groups of fish were similar during the whole period of trial and during the pulse of the functional feeds (0.7% - 0.88% body weight /day). Figure 1 shows the observed survival of the fish the different treatments. Each curve within a subplot denotes a cage.

Figure 1 shows the observed survival of Rainbow trout with an outbreak of SRS given various treatments as described in example 3. Each curve denotes a cage. "Control" is a commercial feed (EWOS Silva), whereas "BG" is a boost premix, and "BG + new" is the boost premix and 15 ppm of Carvacrol and 15 ppm of Thymol.

The estimated hazard ratios in relation to the control feed with approximate 95% credible intervals are given in Table 3.

**Table 3**

| Mean, median and 95% credible interval of the estimated hazard ratios (relative risk of death) in relation to control feed. | | | |
|---|---|---|---|
| Treatment | Mean | Median | 95% credible interval |
| BG | 1.03 | 1.01 | 0.72-1.46 |
| BG + New | 0.81 | 0.79 | 0.57-1.11 |

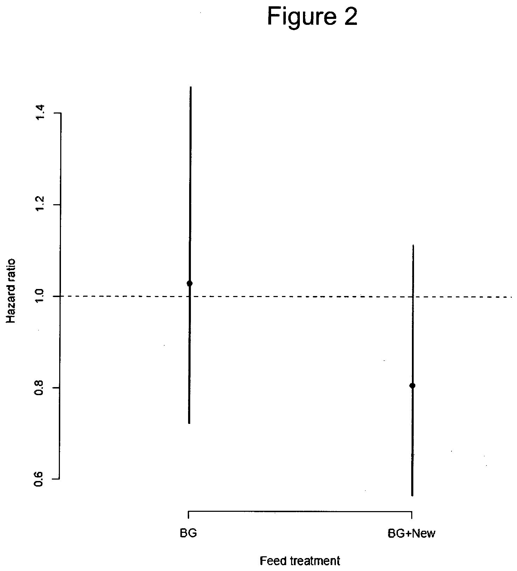

This is also shown graphically in Figure 2 which shows the estimated hazard ratios (relative risk of death) in relation to the control feed (risk taken as 1 and marked with the dashed red horizontal line). Error bars show 95% credible intervals.

Both indicate that the BG+new treatment had decreased the relative risk of mortality by about 20%. The posterior probability of reduced risk of mortality was about 90%. The BG treatment showed a similar hazard ratio to the control feed with a probability of a reducing effect of about 48%.

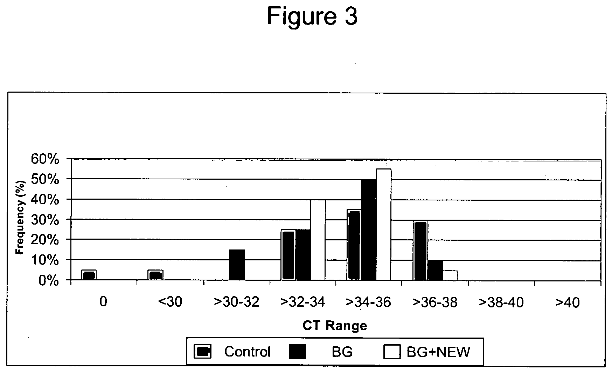

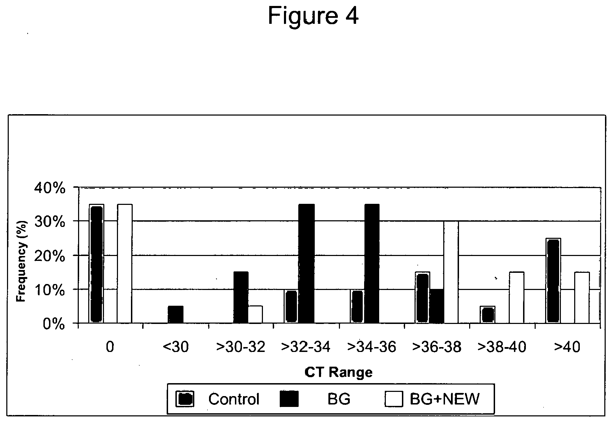

The frequency distributions of CT value for brain tissues analyzed by qPCR are presented in Figure 3 and Figure 4, at the initiation and at the end of the pulse. In the beginning, it was observed a similar distribution of CT values between the treatments (Figure 3), with a mode in the range of 34 to 36. These results confirmed the outbreak of SRS. After 21 days with the different treatments, it was observed that 95% of the fish fed with the BG+New had CT values > 36, compared to 80% for the Control (Figure 4). BG feed fish had lower CT values than the other two treatments. A higher CT value means lower levels of the bacteria in the tissue. Therefore, after 21 of treatments BG+new feed obtained the higher performance to reduce *P. salmonis* comparing with control feed and BG feed.

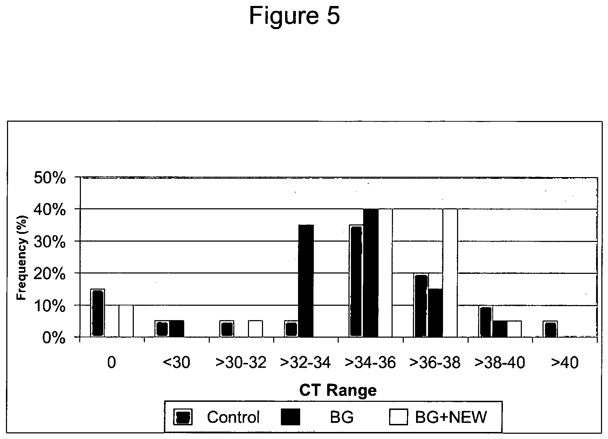

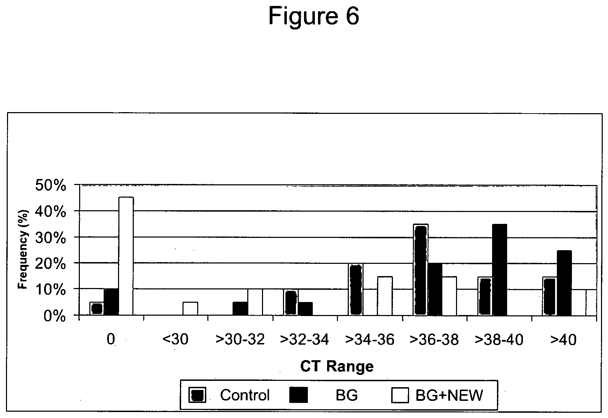

The frequency distributions of CT value for kidney tissues analyzed by qPCR are presented in Figure 5 and Figure 6, at the initiation and at the end of the pulse. In the beginning, it was observed a similar distribution of CT values around 34 to 36 for all treatments (Figure 5). These results confirmed the outbreak of SRS. After 21 days with the different treatments, it was observed that 45% of the fish fed with the BG+New were negative for *P. salmonis.* Moreover, 80% of the fish fed BG had CT values > 36 compared to 65% for the control (Figure 6). Therefore, after 21 of treatments BG+new feed obtained the higher performance to reduce *P. salmonis* comparing with BG feed and control feed.

The following conclusions can be obtained from this study:
- Carvacrol and Thymol added in feed (BG+new) in a dose of 30 ppm (15 ppm of Carvacrol and 15 ppm of Thymol) demonstrated to be more efficient to reduce the mortality of Rainbow trout caused by SRS outbreak than BG feed and control feed.
- Carvacrol and Thymol added in feed (BG+new) in a dose of 30 ppm (15 ppm of Carvacrol and 15 ppm of Thymol) obtained the higher performance to reduce *P.salmonis* comparing with control feed and BG feed.
- A pulse of Carvacrol and Thymol added in feed in a dose of 30 ppm (15 ppm of Carvacrol and 15 ppm of Thymol) for 21 days, can reduce the mortality of Rainbow trout after an outbreak of SRS.

### Definitions used in the application

Propyl-methyl-phenol is a phenolic compound substituted with propyl and methyl on the aromatic ring structure.

Carvacrol is the compound 5-isopropyl-2-methylphenol (C₆H₃CH₃(OH)(C₃H₇)), and is a monoterpenoid phenol. It has a characteristic pungent, warm odor of oregano and a pizza-like taste. It is present in the essential oil of *Origanum vulgare,* oil of thyme, oil obtained from pepperwort, and wild bergamot.

Thymol is the compound 5-isopropyl-2-methylphenol, and is a monoterpenoid phenol derivative of cymene, isomeric with Carvacrol, found in oil of thyme, and extracted as a white crystalline substance of a pleasant aromatic odor and strong antiseptic properties.

## Claims

1. Propyl-methyl-phenol compound, or composition comprising a propyl-methyl-phenol compound for use in the treatment and/or prophylaxis of Infectious salmon anemia (ISA) and/or diseases caused by Infectious salmon anemia virus.

2. Compound for use in accordance with claim 1, wherein said compound is isopropyl-methyl-phenol.

3. Compound for use in accordance with claim 1, wherein said compound is 2-isopropyl-methyl-phenol.

4. Compound for use in accordance with claim 1, wherein said compound is 2-isopropyl-2-methyl-phenol, i.e. Carvacrol.

5. Compound for use in accordance with claim 1, wherein said compound is 2-isopropyl-5-methyl-phenol, i.e. Thymol.

6. Composition for use in accordance with claim 1, wherein the composition comprises a mixture of Carvacrol and Thymol.

7. Composition for use in accordance with claim 1 or 6, wherein the composition comprises a mixture of Carvacrol and Thymol, wherein the concentration is about 100 mg/L applied to an aquatic locus, and about 120 mg/Kg as a component of a feed.

8. Composition for use in accordance with claim 6, wherein the ratio of Carvacrol:Thymol is in the range of 1:3 to 3:1, more preferable 1:2 to 2:1, and most preferable about 1:1, based on weight.

9. Compound or composition for use in accordance with any of the claims 1 to 8, wherein said fish is a Salmonidae.

10. Compound or composition for use in accordance with any of the claims 1 to 9, wherein said fish is selected from the group consisting of Atlantic salmon (*Salmo salar*), Rainbow trout (*Oncorhynchuss mykiss*), Coho salmon (*Oncorhynchus kisutch*) and Artic charr (*Salvelinus alpinus*).

11. Use of a propyl-methyl-phenol compound, or composition comprising a propyl- methyl-phenol compound for the manufacturing of a pharmaceutical and/or nutraceutical composition the treatment and/or prophylaxis of Infectious salmon anemia (ISA) and/or diseases caused by Infectious salmon anemia virus.

## Patentansprüche

1. Propyl-methyl-phenol-Verbindung, oder Zusammensetzung, umfassend eine Propyl-methyl-phenol-Verbindung, zur Verwendung bei der Behandlung und/oder Prophylaxe von Infectious-Salmon-Anemia (ISA) und/oder Krankheiten, die durch den Infectious-Salmon-Anemia-Virus verursacht werden.

2. Verbindung zur Verwendung gemäß Anspruch 1, wobei die Verbindung Isopropyl-methyl-phenol ist.

3. Verbindung zur Verwendung gemäß Anspruch 1, wobei die Verbindung 2-Isopropyl-methyl-phenol ist.

4. Verbindung zur Verwendung gemäß Anspruch 1, wobei die Verbindung 2-Isopropyl-2-methyl-phenol, d. h. Carvacrol, ist.

5. Verbindung zur Verwendung gemäß Anspruch 1, wobei die Verbindung 2-Isopropyl-5-methyl-phenol, d. h. Thymol, ist.

6. Zusammensetzungzur Verwendung gemäß Anspruch 1, wobei die Zusammensetzung eine Mischung aus Carvacrol und Thymol umfasst.

7. Zusammensetzungzur Verwendung gemäß Anspruch 1 oder 6, wobei die Zusammensetzung eine Mischung aus Carvacrol und Thymol umfasst, wobei die Konzentration etwa 100 mg/1 ist, angewandt an einem aquatischen Locus, und etwa 120 mg/kg als eine Komponente eines Futters ist.

8. Zusammensetzung zur Verwendung gemäß Anspruch 6, wobei das Verhältnis von Carvacrol:Thymol, basierend auf dem Gewicht, im Bereich von 1:3 bis 3:1, bevorzugter 1:2 bis 2:1 und am bevorzugtesten bei etwa 1:1 liegt.

9. Verbindung oder Zusammensetzung zur Verwendung gemäß einem der Ansprüche 1 bis 8, wobei der Fisch ein Salmonidae ist.

10. Verbindung oder Zusammensetzung zur Verwendung gemäß einem der Ansprüche 1 bis 9, wobei der Fisch aus der Gruppe ausgewählt ist, die aus Atlantischem Lachs (*Salmo salar*), Regenbogenforelle (*Oncorhynchuss mykiss*), Silberlachs (*Oncorhynchus kisutch*) und Arktischem Saibling (*Salvelinus alpinus*) besteht.

11. Verwendung einer Propyl-methyl-phenol-Verbindung, oder Zusammensetzung umfassend eine Propyl-methyl-phenol-Verbindung, für die Herstellung eines Pharmazeutikums und/oder Nutrazeutikums [für] die Behandlung und/oder Prophylaxe von Infectious-Salmon-Anemia (ISA) und/oder Krankheiten, die durch den Infectious-Salmon-Anemia-Virus verursacht werden.

## Revendications

1. Composé de propyl-méthyl-phénol, ou composition comprenant un composé de propyl-méthyl-phénol pour utilisation dans le traitement et/ou la prophylaxie de l'anémie infectieuse du saumon (ISA) et/ou des maladies causées par le virus de l'anémie infectieuse du saumon.

2. Composé pour utilisation selon la revendication 1, ledit composé étant l'isopropyl-méthylphénol.

3. Composé pour utilisation selon la revendication 1, ledit composé étant 2-isopropyl-méthylphénol.

4. Composé pour utilisation selon la revendication 1, ledit composé étant 2-isopropyl-2-méthylphénol, c'est-à-dire le carvacrol.

5. Composé pour utilisation selon la revendication 1, ledit composé étant le 2-isopropyl-5-méthylphénol, c'est-à-dire le thymol.

6. Composition pour utilisation selon la revendication 1, ladite composition comprenant un mélange de carvacrol et thymol.

7. Composition pour utilisation selon la revendication 1 ou 6, ladite composition comprenant un mélange de carvacrol et thymol, dans laquelle la concentration est d'environ 100 mg/L pour application à un locus aquatique, et d'environ 120 mg/kg en tant que composant d'une alimentation.

8. Composition pour utilisation selon la revendication 6, dans laquelle le rapport carvacrol:thymol est dans la plage de 1:3 à 3:1, de préférence encore 1:2 à 2:1, et idéalement autour 1:1, selon le poids.

9. Composé ou composition pour utilisation selon l'une quelconque des revendications 1 à 8, ledit poisson étant un salmonidé.

10. Composé ou composition pour utilisation selon l'une quelconque des revendications 1 à 9, ledit poisson est sélectionné dans le groupe constitué de : saumon d'atlantique (Salmo salar), truite arc-en-ciel (Oncorhynchus mykiss), saumon coho (Oncorhynchus kisutch) et omble chevalier (Salvelinus alpinus).

11. Utilisation d'un composé de propyl-méthyl-phénol, ou d'une composition comprenant un composé propyl-méthyl-phénol, pour la fabrication d'une composition pharmaceutique et/ou nutraceutique pour le traitement et/ou la prophylaxie de l'anémie infectieuse du saumon (AIS) et/ou des maladies causées par le virus de l'anémie infectieuse du saumon.
